# EUROPEAN PATENT SPECIFICATION

(11) **EP 1 933 809 B1**
(45) Date of publication and mention of the grant of the patent: **08.02.2012**
(21) Application number: 06796170.6
(22) Date of filing: 15.10.2006
(51) Int. Cl.: A61K 9/00, A61K 47/24, A61K 47/10, A61K 47/32, A61K 47/28, A61K 47/26, A61K 47/18, A61K 47/14, A61K 38/28, A61K 38/16, A61K 31/435, A61K 31/5513, A61K 31/335, A61K 31/505, A61K 31/4525

(54) **COMPOSITIONS FOR NASAL DELIVERY**
ZUSAMMENSETZUNGEN FÜR DIE NASALE ABGABE
COMPOSITIONS ADMINISTRABLES PAR VOIE INTRA-NASALE

(30) Priority: 11.10.2005 US 724904 P
(43) Date of publication of application: 25.06.2008
(73) Proprietor: YISSUM RESEARCH DEVELOPMENT COMPANY OF THE HEBREW UNIVERSITY OF JERUSALEM, 91390 Jerusalem (IL)
(72) Inventor: TOUITOU, Elka, 93893 Jerusalem (IL); GODIN, Biana, 96633 Jerusalem (IL); DUCHI, Shaher, 30055 Kfar Rama (IL)
(74) Representative: Vossius & Partner
(86) International application number: PCT/IL2006/001187
(87) International publication number: WO 2007/043057

(56) References cited:
- WO-A-01/06987
- WO-A-03/048167
- US-A- 5 716 638
- US-A1- 2002 048 551
- US-B1- 6 350 458

## Description

Nasal drug delivery is a popular way to treat local/respiratory ailments which has traditionally been restricted to administer drugs for sinus conditions, such as congestion and allergies. Recently, however, there has been increased interest in the nose as an alternative to oral and parenteral delivery for many systemic drugs and vaccines. The vastly vascularised and immunogenic nasal mucosa present potential benefits for systemic absorption in terms of quick action, avoidance of any degradation and/or unwanted entero-hepatic metabolism of the drug (improved bio-availability) and patient compliance as well as improved immune response for vaccines. The nasal route could also provide an attractive needle-free alternative for currently injectable drugs which may improve patient compliance and allow extended use of self-medication for many chronic diseases/acute conditions or vaccinations. Some systemically-acting drugs for the treatment of osteporosis, cardiovascular medications and painkillers are already on the market in nasal formulations.

However, although this route is beginning to be explored for systemic delivery of drugs the major limitation in nasal delivery is the insufficient permeation of drugs across the nasal mucosa. Furthermore, the anatomical and physiological features of the nose are not ideal for drug administration, since a relatively small surface area (150 cm²) puts considerable constraints on formulations and drug candidates. Only very potent molecules can be used in this route. For example, for peptides there is the inverse relationship between bioavailability and molecular weight of the peptide which points toward, that those peptides with more than 30-40 amino acids require penetration enhancers for attaining a sufficient bioavailability (in the range of 10%). There are two main pathways for absorption of the molecule from the nasal cavity: paracellular (driven by passive diffusion) or transcellular (driven by carrier or receptor mediated active transport). In the absence of active transport components, most peptides cross the nasal epithelium by the paracellular route, driven by passive diffusion. Due to hydrophilicity of peptides the transcellular route is mainly relevant for transport processes or for transcytosis. Both transcellular routes are energy dependent and are therefore designated as active transport processes.

The issue of improving nasal absorption is important. Several strategies have been investigated in the past decade such as chelators of calcium (EDTA), inhibition of nasal enzymes (boro-leucin, aprotinin), inhibition of muco-ciliar clearance (preservatives), solubilisation of nasal membrane (cyclodextrin, fatty acids, surfactants) and formation of micelles (surfactants). Many surfactants such as bile acids, Laureth 9 and taurodehydrofusidate (STDHF) turned out to be quite effective in enhancing nasal absorption, but caused local cytotoxic effects on ciliated cells. Therefore, enhancers with an acceptable safety profile under chronic treatment are still to be discovered. A greater permeability of drug through nasal mucosa has the potential to overcome the limitations of oral route and to approach the benefits of intravenous infusion. Safe and efficacious enhancers will be necessary for commercially successful products.

The delivery of biologically active materials to the skin and cell membranes by means of an aqueous vehicle that comprises the combination of lipid vesicles and water miscible organic solvents has been described in the art.

For example, an aqueous carrier system containing phospholipids and ethanol was described in EP 158441, with the weight ratio between the aforementioned components being from 40:1 to 1:20.

US 2002/0048551 A1 discloses compositions for nasally administering agents comprising by weight percent 0.25 to 20% lecithin and 0.1 to 10% ethanol.

US 5,711,965 describes a solution comprising phospholipids, ethanol and water in a weight ratio of 10:16:74, respectively.

US 5,540,934, US 5,716,638 and WO 03/000174 describe an aqueous composition containing vesicles (ethosomes) in the presence of ethanol.

US 6,627,211 describes a carrier suitable for the administration of an anti-convulsive agent to the nasal mucous membranes. It appears that the content of organic solvents in said carrier is relatively high (30% to 60% ethanol and 30 to 60% propylene glycol).

It has now been found that an aqueous composition which contains phospholipids in a concentration of 0.2 to 10% by weight, in combination with one or more short chain alcohols, wherein the weight concentration of water is not less than 30% by weight and the weight concentration of said alcohol(s) is in the range between 12 to 30% by weight, may be adapted for use as an intranasal drug delivery vehicle.

Accordingly, the present invention provides the use of phospholipid, one or more C2-C4 alcohols and water in the preparation of a vesicular composition adapted for intranasal administration of an active agent, wherein the concentrations of said phospholipid and said one or more alcohols in said composition are in the ranges of 0.2 to 10% and 12 to 30% by weight, respectively, and the water content of said composition is not less than 30% by weight.

Preferably, the water content in the composition is not less than 35%, and more preferably not less than 45%. The weight ratio between the alcohol(s) and the phospholipids is not less than 2:1, and more preferably not less than 5:1.

Phospholipids suitable for use in the preparation of the composition according to the present invention include phosphatidylcholine (PC), hydrogenated phosphatidylcholine, phosphatidic acid (PA), phosphatidylserine (PS), phosphatidylethanolamine (PE), phosphatidylglycerol (PPG) and phosphatidylinositol (PL). The chemical structure of phospholipids that may be used according to the present invention is described in US 4,614,730. Preferably, the phospholipids are present in the composition of the invention at a concentration of 0.5 to 5% by weight.

The term C2-C4 alcohols, as used herein, refers to alkanols containing two, three or four carbon atoms. The alcohols to be used according to the present invention specifically include ethanol, 1-propanol, isopropyl alcohol and tert-butyl alcohol, with the former being especially preferred. The concentration of ethanol in the composition contemplated by the present invention for use as an intranasal drug delivery vehicle is preferably in the range of 15 to 27% by weight.

According to a particularly preferred embodiment of the invention, the composition further comprises one or more water miscible polyols, and especially glycols (1,2-diols, such as ethylene glycol and propylene glycol; with the latter being especially preferred), at a concentration of 1 to 30% by weight, and preferably 5 to 20 by weight.

The compositions of the present invention may be prepared by mixing together the various components, namely, water, phospholipids, one or more C2-C4 alcohols (and possibly also one or more polyols) and the active ingredient under conditions that allow the formation of vesicles. More specifically, the compositions of the present invention may be conveniently prepared by dissolving the phospholipids in the alcohol (or in the alcohol/glycol mixture), followed by the addition of the active ingredient, either in the form of an aqueous solution thereof or in a solid form, with a subsequent addition of water. The preparation of the composition is preferably carried out under stirring, typically at room temperature or at an elevated temperature, which is preferably not higher than 50°C.

Alternatively, a dispersion of the phospholipids and the active ingredient in water is prepared, into which the alcohol, optionally together with polyol (e.g., a mixture of ethanol and propylene glycol) are added with stirring, possibly under heating.

It is also possible to first prepare freeze-dried lipid vesicles having the active ingredient encapsulated therein, and subsequently dispersing the same in a mixture of water, the C2-C4 alcohol and optionally polyol.

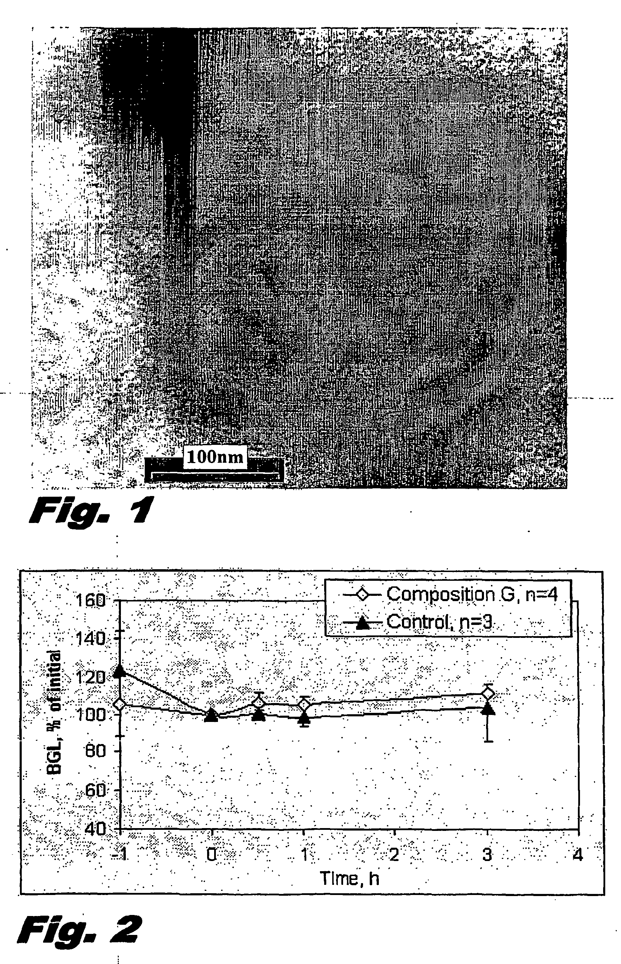

As mentioned above, the combination of phospholipids, water, and the water-miscible organic solvents (namely, the alcohol and the polyol) according to the concentrations and weight ratios specified above allows the formation of a non-irritant, vesicular composition, with the vesicles present therein, whose size ranging between 50 nm to few microns, and more specifically, up to 5µm, exhibiting good properties for enhanced nasal absorption. Figure 1 is TE (transmission electron) micrograph of a specific composition according to the present invention (containing insulin as the active agent; the exact composition is given in the Examples below - entry F in table 1A). It may be seen that in this specific system, the vesicular structures are multilamellar. The vesicles were visualized by transmission electron microscopy (TEM) and scanning electron microscopy. TEM analysis was carried out using a Philips TEM CM 12 electron microscope (TEM, Eindhoven, The Netherlands) with an accelerating voltage of 100kV.

Thus, the present invention concerns methods for intranasal administration, and compositions for intranasal administration comprising vesicular systems formed from at least one active molecule, phospholipid, alcohol (C2-C4) and water. Optionally, the composition further comprises glycol (propylene glycol, transcutol, tetraglycol, etc).

We have found that pharmaceutical formulations including the above ingredients could deliver therapeutic amounts of agents to the systemic circulation or the brain of mammals and have efficient therapeutic or prophylaxis effect. The invention can be used for pharmaceutical, cosmetic, medical, veterinary, diagnostic and research applications. The present invention includes nasally administering to the mammal a therapeutically effective amount of active ingredient by means of compositions described above. The nasal delivery may be either for local purposes (to the mucosa of the nose), for systemic administration through the circulation or for CNS administration for curing brain disease.

It should be noted that the composition according to the present invention may include additional excipients that are well known in the art, such as surfactants, preservatives, thickening agents, co-solvents, adhesives, antioxidants, buffers, viscosity and absorption enhancing agents and agents capable of adjusting the pH and osmolarity of the formulation.

Suitable surfactants that can be used in accordance with the present invention include ionic, nonionic or amphoteric surface active agents. More specifically, hydrophilic surfactants (e.g. Tweens, Tween 80, Myrj, Brjs, Labrasol etc.) or lipophilic surfactants (eg. Span 20, Span 60, Myrj, Arlacel 83 and such) may be suitably used, preferably at a concentration in the range of 0-25% by weight.

Suitable preservatives that can be used with the present formulations include, for example, benzyl , alcohol, parabens, chlorobutanol, benzalkonium salts and combinations thereof. Some examples of antioxidants include tocopherols, butyl hydroxytoluene, sodium metabisulfite, potassium ..metabisulfite, ascorbyl palmitate and the like. These preservatives and antioxidants may be present in the formulations in a concentration of from about 0.001% up to about 5%w/w.

Regarding buffers, the nasal delivery system may include a buffer for maintaining the formulation at a pH of about 7.0. The particular buffer, of course, can vary depending upon the particular nasal delivery system used, as well as the specific active molecule selected. Buffers that are suitable for use in the present invention include, for example, acetate, citrate, prolamine, carbonate and phosphate buffers and combinations thereof. The pharmaceutical formulations of the present invention may include a pH adjusting agent.

Regarding thickening agents, the viscosity of the formulations of the present invention can be maintained at a desired level using a pharmaceutically acceptable thickening agent. Thickening agents that can be added to the compositions of the present invention include for example, methyl cellulose, xanthan gum, tragacanth, adhesives, guar gum, carboxymethyl cellulose, hydroxypropyl cellulose, carbomer, polyvinyl alcohol, alginates, acacia, chitosans, mucoadhesive polymer-systems like poly(acrylates), cellulose derivatives, hyaluronic acid, hyaluronic acid derivatives, chitin, collagen, pectin, starch, poly(ethylene glycol), sulfated polysaccharides, carrageenan, Na-alginate, gelatine, pectin and combinations thereof. The desired concentration of the thickening agent will depend upon the agent selected and the viscosity desired.

The compositions may also comprise gel forming or bioadhesive compounds such as carbopols, alginates, scleroglucan, cellulose derivatives, starch, albumin, pluronic gels, diethyl aminoethyl (DEAE)-sephadex, polycarbophil, hyaluronic acid, hyaluronates, starch, gelatin, cholagen and others. Compositions can also be incorporated in the w/o cream, o/w cream, hydrophilic ointment or lipophilic ointment, gels, other semi-solid bases. The compositions could be delivered to the nasal cavity as drops, mists, aerosols, instillations, by use of pipetor, special devices, evaporators, vaporizators and such.

The formulations of the present invention may also include agents such as tolerance enhancers to reduce or prevent drying of the mucus membrane and to prevent irritation thereof.

The compositions according to the present invention may be applied to the nasal cavity as liquids, sprays, aerosols, nebulizaers or semi-solid preparations. Semisolid preparations may be on the base of gels, w/o or o/w creams or hydrophilic/lipophilic ointments. The compositions may contain molecularly dispersed (soluble, solubilized, etc.) active agent or the fine particles/crystals of the active agent. The compositions could be administered from nasal sprays, metered-dose sprays, squeeze bottles, liquid droppers, disposable one-dose droppers, nebulizers, cartridge systems with unit-dose ampoules, single-dose pumps, bi-dose pumps, multiple-dose pumps or any other device. For example, the compositions of the invention may be stored in/delivered from a spray or aerosol device/container as described in details in Remington's Pharmaceutical Sciences (16th edition, Chapters 83 and 92).

Regarding spray devices, it should be noted that both single (unit) dose or multiple dose systems may be used. Typically, a spray device comprises a bottle and a pump; such devices are commercially available from various sources. Typically, the volume of liquid that is dispensed in a single spray actuation is in the range of from 5 to 250 microliters/each nostril/single administration and the concentration of the active ingredient in the formulation may be readily adjusted such that one or more spray into the nostrils will comply with the dosage regimen.

Also a spray device or a disclosed are dose cartridge for use in a nasal delivery device loaded with a composition as described above.

Also disclosed is a method of administering an active pharmaceutical ingredient to a patient in need thereof, which method comprises the intranasal administration of a vesicular composition comprising a therapeutically effective amount of said ingredient, phospholipids, one or more C2-C4 alcohols and water, wherein the concentrations of said phospholipids and said one or more alcohols in said composition are in the ranges of 0.2 to 10% and 12 to 30% by weight, respectively, with the water content of said composition being not less than 20%, and preferably not less than 30% by weight.
Mammals include humans, pet animals, laboratory animals, farm animals and wild animals.

The intranasal drug delivery vehicle according to the present invention may be adapted for the administration of active agents that can be used for medical, pharmaceutical, veterinary, research or diagnostic purposes. However, especially preferred active agents to be used according to the present invention include an anti-diabetic agent (e.g., insulin or derivative thereof), an anti-malaria agent (which is most preferably dihydroartemisinin); an anti-anxiety agent and an anticonvulsant (which is most preferably diazepam) and anti-emetic agent (which is most preferably granisetron hydrochloride); an anti-anxiety/anti-depressant (which is most preferably buspirone hydrochloride); an anti-multiple sclerosis agent (which is most preferably glatiramer acetate); an anti-depressant/ an anti-hot flashes agent (which is most preferably paroxetine or a pharmaceutically acid addition salt thereof); an anti-dementia/Alzheimer's agent (which is most preferably rivastigmine); and an anti-obesity agent (which is most preferably sibutramine).

More specifically, it has now been found that the intranasal drug delivery vehicle according to the present invention may be used for the intranasal administration of insulin. The term insulin or derivative thereof, as used herein, encompasses rapid acting (e.g. insulin aspart, insulin glulisine, insulin lispro), short-acting (regular), intermediate-acting (NPH), intermediate and short acting mixtures and long-acting insulin (e.g. insulin glargine, insuline detemir) (according to FDA classification as appears in www.fda.gov/fdac/features/2002/chrt_insulin.html).
Insulin is typically administered at daily dose of 1.5 to 150IU.

Accordingly, in another aspect, the present invention provides a pharmaceutical composition for intranasal administration, which comprises a therapeutically effective amount of insulin or a derivative thereof together with water, phospholipids and one or more C2-C4 alcohols, wherein the concentrations of said phospholipids and said one or more alcohols are in the ranges of 0.2 to 10% and 12 to 30% by weight, respectively, with the water content of said composition being not less than 30% by weight. Preferably, the composition further comprises a polyol, and more specifically, propylene glycol, at a concentration in the range of 1 to 30% by weight.

Also disclosed is a method for treating diabetes in a mammal, which method comprises the intranasal administration of the aforementioned insulin-containing composition.

It has now been also found that the intranasal drug delivery vehicle according to the present invention may be used for the intranasal administration of diazepam. Diazepam is 7-chloro-1,3-dihydro-1-methyl-5-phenyl-2H-1,4-benzo-diazepin-2-one. A method for the synthesis of diazepam has been described, for example by Sternbach LH, Reeder E, Keller O, & Metlesics W. [Quinazolines and 1,4-benzodiazepines III substituted 2-amino-5-phenyl-3H-1,4-benzodiazepine 4-oxides. J Org Chem, 26: 4488-4497, 1961]. Diazepam is typically administered at a daily dose of 0.2 to 100 mg.

Accordingly, in another aspect, the present invention provides a pharmaceutical composition, which comprises a therapeutically effective amount of diazepam together with water, phospholipids and one or more C2-C4 alcohols, wherein the concentrations of said phospholipids and said one or more alcohols are in the ranges of 0.2 to 10% and 12 to 30% by weight, respectively, with the water content of said composition being not less than 30% by weight. Preferably, the composition further comprises a polyol, and more specifically, Propylene glycol, at a concentration in the range of 1 to 30% by weight.

Also disclosed is a method for preventing and/or treating epileptic seizures in a mammal, which method comprises the intranasal administration of the aforementioned diazepam-containing composition.

It has now been also found that it is possible to prepare a pharmaceutical composition of Granisetron [an anti-emetic agent, which is chemically named: endo-1-methyl-N-(9-methyl-9-azabicycle[3.3.1]non-3-yl)-1H-indazole-3-carboxamide] that is suitable for the intranasal administration of said drug. Granisetron is described in EP 200444; methods for preparing granisetron are also described in WO03/080606. Granisetron is typically administered at a daily dose of 0.1 to 10 mg.

Accordingly, in another aspect, the present invention provides a pharmaceutical composition, which comprises a therapeutically effective amount of granisetron or a pharmaceutically acceptable salt thereof together with waster, phospholipids and one or more C2-C4 alcohols, wherein the concentrations of said phospholipids and said one or more alcohols are in the ranges of 0.2 to 10% and 12 to 30% by weight, respectively, with the water content of said composition being not less than 30% by weight. Preferably, the composition further comprises a polyol, and more specifically, propylene glycol, at a concentration in the range of 1 to 30% by weight.

Also disclosed is a method for treating and/or preventing emesis in a mammal, which method comprises the intranasal administration of the aforementioned granisetron-containing composition.

Other compositions for intranasal administration contemplated by the present invention comprise:
(i) a therapeutically effective amount of an a pharmaceutically active ingredient selected from the group consisting of buspirone, glatiramer, paroxetine, rivastigmine and sibutramine and a pharmaceutically acceptable salt thereof, together with:
(ii) water;
(iii) phospholipids; and
(iv) one or more C2-C4 alcohols;
wherein the concentrations of said phospholipids and said one or more alcohols are in the ranges of 0.2 to 10% and 12 to 30% by weight, respectively, with the water content of said composition being not less than 30% by weight. Preferably, the composition further comprises a polyol, and more specifically, propylene glycol, at a concentration in the range of 1 to 30% by weight.

Also disclosed is a method for preventing and/or treating obesity in a mammal, which method comprises the intranasal administration of the aforementioned sibutramine-containing composition. Sibutramine is typically administered at a daily dose of 1 to 30 mg. Its preparation is described by Jeffery et al., [Synthesis of Sibutramine, A Novel Cyclobutylalkylamine Useful in the Treatment of Obesity and its Major Human Metabolites, J. Chem. Soc. Perkin. Trans. 1, 2583-2589 (1996)] and also in US Patent Nos. 4,746,680; 4,929,629; and 5,436,272.

Also disclosed is a method for preventing and/or treating dementia, and specifically, Alzheimer disease in a mammal, which method comprises the intranasal administration of the aforementioned rivastigmine-containing composition. Rivastigmine may be administered as its hydrogen tartrate salt at a daily dose of 1 to 20 mg.

Also disclosed is a method for treating multiple sclerosis in a mammal, which method comprises the intranasal administration of the aforementioned glatiramer-containing composition. Glatiramer is typically administered at a daily dose of 1 to 60 mg. Glatiramer acetate is a mixture of polypeptides composed of alanine, glutamic acid, lysine, and tyrosine in a molar ratio of approximately 4.6:1.5:3.6:1.0, respectively, which is synthesized by chemically polymerizing the four amino acids, forming products with average molecular weights ranging from about 4000 to about 13,000 daltons. The corresponding molar fractions are approximately 0.427 for alanine, 0.141 for glutamic acid, 0.337 for lysine and 0.093 for tyrosine, and may vary by about +/-10%.

Also disclosed is a method for treating depression and/or hot flushes in a mammal, which method comprises the intranasal administration of the aforementioned paroxetine-containing composition. Paroxetine is typically administered at a daily dose of 5 to 100 mg. Its preparation is described, for example, in US 6,956,121 and US 6,686,473.

An especially important aspect is related to the treatment of malaria. In malaria prevalent regions of the world, Plasmodium infections is the reason for a very high mortality rates (hundreds of thousands of deaths), especially among children. Many patients with acute malaria are unable to tolerate oral therapy and parenteral treatment, which could only be available at hospitals, is necessary. However, these amenities are usually inaccessible.

It has now been found that anti-malaria drug administered intranasally is effective at least as or even more that i.p. administration. This finding paves the way to the formulation of a pharmaceutical composition for intra-nasal administration comprising a carrier and at least one anti-malaria agent.

Examples of anti-malaria drugs are artemisinin derivatives, dihydroartemisinin, artemotil, chloroquine, primaquine, doxycillin, quinine, aminoquinolines, cinchona alkaloids, antifolates, quinidine, melfoquine, halofantrine, lumefantrine, amodiaquine, pyronaridine, tafenoquine, artesunates, artemether, artemotil, biguanides, proguanil, chloproguanil, diaminopyrimidines, pyremethamine, trimethoprim, dapsone, sulfonamides, atovaquone, sulfadoxine-pyrimethamine, N-acetyl cysteine, piperaquine, DHA-piperaquine, lumefantrine, dermaseptins, bisphosphonates, quercitin etc.

Also disclosed is a pharmaceutical composition for intra-nasal administration comprising a carrier and at least one anti-malaria drug, wherein said carrier is most preferably a vesicular carrier (namely, a carrier that contain vesicles suspended therein), and also with the use of an anti-malaria agent in the preparation, of a medicament for intra-nasally treating malaria.

An intranasal composition may comprise any carrier or combination of carriers known to be suitable for intranasal administration. The composition may comprise at least one anti malaria agent in combination with the intranasal drug delivery vehicle as described above, which vehicle comprises not less than 30% by weight water, from 12 to 30% by weight C2-C4 alcohol(s), from 1 to 30% by weight water-miscible polyol(s), from 0.2 to 10% phospholipids arranged in a vesicular structure. Other features of the anti-malaria composition are as described above in connection with said intranasal drug delivery vehicle.

Also disclosed is a method for treating malaria (including cerebral malaria) comprising: administering intra-nasally to a subject in need of such treatment a therapeutically effective amount of at least one anti-malaria drug. The anti-malaria drug may be dihydroartemisinin, which is typically administered at the following dosage regimen: Adults: 40-120mg/day in divided doses for 6-7 days; Children: 2-4 mg/kg in a divided loading dose on the first day followed by 1-2 mg/kg daily for 6 days. Dihydroartemisinin can be prepared by reduction of artemisinin with sodium borohydride; [A. Brossi et al., Arteether, a New Antimalarial Drug: Synthesis and Antimalarial Properties, J. Med. Chem. 31, 645-650 (1988)].

As used herein, nasally administering or nasal administration includes administering the compositions into naristilles of the nose to the mucous membranes of the nasal passage or nasal cavity of the mammal. Such formulations can be administered, for example, as a nasal spray, nasal inhaler, nasal drop, aerosol, propellants, pressured dispersion, aqueous aerosol, nebulizer, nasal suspension, instillation, nasal gel, nasal ointment and nasal cream by aid of any new or old type device. Administration of compositions of the present invention may also take place using a nasal tampon or nasal sponge containing the compositions.

Active ingredient can also be brought into a viscous base by adding to the above delivery systems conventionally used ingredients such as natural gums, cellulose and derivatives, acrylic polymers (eg.carbopol) and vinyl polymers (polyvinylpyrrolidone), scleroglucans, xylan, alginates, calcium alginate, hyaluronates, collagenates, starch gells, gelatine systems, kitosan carriers.

It should be understood that the intranasal drug delivery vehicle according to the present invention is not limited for the administration of the specific active ingredients mentioned above. It should be noted that the active agent can be a chemically defined synthetic molecule, a naturally derived or synthetic peptide, a protein, a polysaccharide, or a nucleic acid such as RNA or DNA. The active agent may also be referred to as active compound, drug, drug substance, medicinal substance, therapeutic agent, and the like. The active agents that could be delivered by means of the above compositions alone or in combinations are without being limited:
- Antimalarial agents (e.g. artemisinin derivatives, dihydroartemisinin, artemotil, chloroquine, primaquine, doxycillin, quinine, aminoquinolines, cinchona alkaloids, antifolates, quinidine, melfoquine, halofantrine, lumefantrine, amodiaquine, pyronaridine, tafenoquine, artesunates, artemether, artemotil, biguanides, proguanil, chloproguanil, diaminopyrimidines, pyremethamine, trimethoprim, dapsone, sulfonamides, atovaquone, sulfadoxine-pyrimethamine, N-acetyl cysteine, piperaquine, DHA-piperaquine, lumefantrine, dermaseptins, bisphosphonates, quercitin etc. The drugs could be used alone or in combinations.)
- OTC drugs (e.g. antipyretics, anesthetics, cough suppressants, etc.)
- Antiinfective agents
   Anti-malaria agents (such as dihydroartemisinin, etc.)
- Antibiotics (e.g. penicillins, cephalosporins, macrolids, tetracyclines, aminoglycosides, antituberculosis agents, doxycycline, ciprofloxacine, moxifloxacine, gatifloxacine, carbapenems, azithromycine, clarithromycine, erythromycine, ketolides, penems, tobramyicin, filgrastim, pentamidine, microcidin, clerocidin; amikacine, etc.)
- Antifungal/Antimycotic (metronidazole, ketoconazole, itraconazole, voriconazole, clotrimazole, bifonazole, fluconazole, amphotericine B, natamycine, nystatine, ciclopiroxolamine, etc.)
- Genetic molecules (e.g. Anti-sense oligonucleotides, nucleic acids, oligonucleotides, DNA, RNA,
- Anti-cancer agents (e.g. anti-proliferative agents, anti-vascularization agents, taxol, etopside, cisplatin, etc.)
- Anti-protozoal agents
- Antivirals (e.g. acyclovir, gancyclovir, ribavirin, anti-HIV agents, anti-hepatitis agents, famciclovir, valaciclovir, didanosine, saquinavir, ritonavir, lamivudine, stavudine, zidovudine, etc.)
- Anti-inflammatory drugs (e.g. NSAIDs, steroidal agents, cannabinoids, leukotriene-antagonists, tacrolimus, sirolimus, everolimus, etc.)
- Anti-allergic molecules (e.g. antihistamines, fexofenadine)
- Bronchodilators
- Vaccines and other immunogenic molecules (e.g. tetanus toxoid, reduced diphtheria toxoid, acellular pertussis vaccine, mums vaccine, smallpox vaccine, anti-HIV vaccines, hepatitis vaccines, pneumonia vaccines, influenza vaccines, TNF-alpha-antibodies etc.)
- Anesthetics, local anesthetics.
- Antipyretics (e.g. paracetamol, ibuprofen, diclofenac, aspirin, etc.)
- Agents for treatment of severe events such cardiovascular attacks, seizures, hypoglycemia, etc.
- Afrodisiacs from plants or synthetics
- Anti-nausea and anti-vomiting.
- Immunomodulators (immunoglobulins, etc.)
- Cardiovascular drugs (e.g. beta-blockers, alpha-blockers, calcium channel blockers, etc.)
- Peptide and steroid hormones (eg. insulin, insulin derivatives, insulin, detemir, insulin monomeric, oxytocin, LHRH, LHRH analogues, adreno-corticotropic hormone, somatropin, leuprolide, calcitonin, parathyroid hormone, estrogens, testosterone, adrenal corticosteroids, megestrol, progesterone, sex hormones, growth hormones, growth factors, etc.)
- Peptide and protein related drugs (e.g. amino acids, peptides, polypeptides, proteins)
- Vitamins (e.g. Vit A, Vitamins from B group, folic acid, Vit C, Vit D, Vit E, Vit K, niacin, derivatives of Vit D, etc.)
- Autonomic.Nervous System Drugs
- Fertilizing agents
- Antidepressants (e.g. buspirone, venlafaxine, benzodiazepins, selective serotonin reuptake inhibitors (SSRIs), sertraline, citalopram, tricyclic antidepressants, paroxetine, trazodone, lithium, bupropion, sertraline, fluoxetine, etc.)
- Agents for smoking cessation (e.g. bupropion, nicotine, etc.)
- Agents for treating alcoholism and alcohol withdrawal
- Lipid-lowering agents (eg. inhibitors of 3 hydroxy-3-methylglutaryl-coenzyme A (HMG-CoA) reductase, simvastatin, atrovastatin, etc.)
- Drugs for CNS or spinal cord (benzodiazepines, lorazepam, hydromorphone, midazolam, Acetaminophen, 4'-hydroxyacetanilide, barbiturates, anesthetics, etc.)
- Anti-epilepsic agents (e.g. valproic acid and its derivatives, carbamazepin, etc.)
- Angiotensin antagonists (e.g. valsartan, etc.)
- Anti-psychotic agents and anti-schizophrenic agents (e.g. quetiapine, risperidone)
- Agents for treatment of Parkinsonian syndrome (e.g. L-dopa and its derivatives, trihexyphenidyl, etc.)
- Anti-Alzheimer drugs (e.g. cholinesterase inhibitors, galantamine, rivastigmine, donepezil, tacrine, memantine, N-methyl D-aspartate (NMDA) antagonists).
- Agents for treatment of non-insulin dependent diabetes (e.g. metformine,
- Agents against erectile dysfunction (e.g. sildenafil, tadalafil, papaverine, vardenafil, PGE1, etc.)
- Prostaglandins
- Agents for bladder dysfunction (e.g. oxybutynin, propantheline bromide, trospium, solifenacin succinate etc.)
- Agents for treatment menopausal syndrome (e.g estrogens, non-estrogen compounds, etc.)
- Agents for treatment hot flashes in postmenopausal women
- Agents for treatment primary or secondary hypogonadism (e.g. testosterone, etc.)
- Cytokines (e.g. TNF, interferons, IFN-alpha, IFN-beta, interleukins etc.)
- CNS stimulants
- Muscle relaxants
- Anti paralytic gas agents
- Appetite stimulators/depressors (e.g. cannabinoids, etc.)
- Gastrointesinal absorption modifiers
- Narcotics and Antagonists (e.g. opiates, oxycodone etc.)
- Painkillers (opiates, endorphins, tramadol, codein, NSAIDs, gabapentine etc.)
- Hypnotics (Zolpidem, benzodiazepins, barbiturates, ramelteon, etc.)
- Histamines and Antihistamines
- Antimigraine Drugs (e.g. imipramine, propranol, sumatriptan, eg.)
- Diagnostic agents (e.g. Phenolsulfonphthalein, Dye T-1824, Vital Dyes, Potassium Ferrocyanide, Secretin, Pentagastrin, Cerulein, etc.)
- Topical decongestants or anti-inflammatory drugs
- Anti-acne agents (e.g. retinoic acid derivatives, doxicillin, minocyclin, etc.)
- ADHD related medication (e.g. methylphenidate, dexmethylphenidate, dextroamphetamine, d- and 1-amphetamin racemic mixture, pemoline, etc.)
- Diuretic agents
- Anti-osteoporotic agents (e.g. bisphosphonates, aledronate, pamidronate, tirphostins, etc.)
- Drugs for treatment of asthma
- Anti-Spasmotic agents (e.g. papaverine, etc.)
- Agents for treatment of multiple sclerosis and other neurodegenerative disorders (eg. mitoxantrone, glatiramer acetate, interferon beta-la, interferon beta-1b, etc.)
- Plant derived agents from leave, root, flower, seed, stem or branches extracts.

### In the drawings

Figure 1 is a TE micrograph of insulin vesicles in a Composition.F according to the invention.
Figure 2 is a graph showing Blood glucose levels (% of initial) in mice following intranasal administration of 25µL of insulin composition G (aqueous control containing 58IU/ml) versus untreated mice.
Figure 3 is a graph showing Blood glucose levels (% of initial) in mice following intranasal administration of 25µL of human insulin compositions C (a composition of the invention containing 58IU/ml insulin) and D (placebo) versus untreated mice.
Figure 4 is a graph showing Blood glucose levels (% of initial) in mice following intranasal administration of 25µL of insulin composition F (a composition of the invention containing 20IU/ml insulin) versus untreated mice.
Figure 5 is a graph showing Blood glucose levels (% of initial) in mice following intranasal administration of 25µL of insulin compositions N and O (compositions of the invention containing 58IU/ml insulin) versus untreated mice.
Figure 6 is a bar diagram showing the results of Writhing test in mice following administration of diazepam vesicular composition prior to writhing induction with acetic acid versus untreated control.
Figure 7 is a bar diagram showing the results of Writhing test in mice following administration of diazepam vesicular carrier(drug dose 5mg/kg) simultaneously with writhing induction with acetic acid solution versus untreated control.
Figure 8 is a bar diagram showing the results of Writhing test in mice following intranasal (IN) administration of diazepam phospholipid ethanolic vesicles Composition (5mg/kg) and subcutaneous (SC) injection of diazepam simultaneously with writhing induction with acetic acid solution versus untreated control.
Figure 9 is a graph depicting the changes in the weight of rats following administration of ipecac syrup and inducing Pica syndrome on day 3. Animals intranasally treated with granisetron HCl Composition B (IN-GR, 1.5mg drug/kg rat, n=5) versus untreated control (n=5).
Figure 10 is a graph showing the changes in the food consumption in rats following administration of ipecac syrup and inducing Pica syndrome on day 3. Animals intranasally treated with granisetron HCl Composition B (IN-GR, 1.5mg drug/kg rat, n=5) versus untreated control (n=5).
Figure 11 is a graph showing the changes in the kaolin consumption in rats following administration of ipecac syrup and inducing Pica syndrome on day 3. Animals intranasally treated with granisetron HCl Composition B (IN-GR, 1.5mg drug/kg rat, n=5) versus untreated control (n=5). '
Figure 12 is a CLS (confocal laser scanning) micrograph showing the transport of Rhodamine B across the nasal mucosa from the composition of the invention applied for 0.5h to the rat nostril. White means the highest fluorescent intensity.
Figure 13 is a graph showing Blood glucose levels (% of initial) in mice following intranasal administration of 25µL of insulin compositions in a comparative study. The concentration of human insulin in all Compositions is 63 IU/mL. Composition I is a composition of the invention; Composition II is a control composition having only 10% EtOH; Composition III is a liposomal control composition.

### Examples

### Materials

Insulin solution used for preparation of the Compositions C-V- is Biosynthetic Human Insulin aqueous solution 100IU/mL (Actrapid, Novartis).

### Example 1

### Insulin-containing composition

20 mg of phospholipids (Phospholipon 90, Natterman were dissolved in 0.3g ethanol (J.T. Baker) and to this solution 0.1g propylene glycol was added. The obtained solution was added slowly to the 0.58 g of the aqueous solution of human insulin (100IU/mL) under constant stirring at room temperature. The composition is stirred for additional 5 min. It is also possible to introduce the aqueous human insulin solution into the phospholipid solution in ethanol and propylene glycol. The final composition contains 58 IU insulin/ g.

### Example 2

### Insulin-containing composition

15 mg of phospholipids (Phospholipon 90) were dissolved in a mixture of 225mg ethanol and 75mg propylene glycol. To the obtained solution, 685 mg of aqueous solution of insulin (100IU/mL) were added slowly under constant stirring at 40C temperature. The composition is stirred for additional 5 min. The final composition contains 68.5 IU insulin/g. This composition is also prepared at room temperature.

### Example 3

### Insulin-containing composition

To -freeze-dried liposomes containing 40 mg phospholipid and 116 IU human insulin a mixture of 0.6g EtOH, 0.2g PG and 1.16g DDW was added in aliquots under constant stirring at room temperature. The composition is stirred for additional 5 min. The final composition containes 58IU insulin/ g (1.45 IU insulin/25 microlitter).

### Example 4

### Insulin-containing composition

To a liposomal dispersion containing 30mg phospholipid, 137 IU insulin and 685mg DDW, 225mg EtOH and 75mg Propylene glycol were added under constant stirring at room temperature. The composition is stirred for additional 5 min. The final composition contains 68.5IU insulin/g.

### Example 5

### Insulin-containing composition

0.05g Carbopol 974P was dispersed in 1mL of insulin aqueous solution (100IU/mL). In a separate container 0.5 g of Phospholipon 90 and 0.15g cholesterol were dissolved in 1.85g ethanol and to this solution 0.95g propylene glycol were added. To this mixture 0.65g Tween 20 were added. To the obtained system 4.8mL of insulin aqueous solution (100IU/mL) were added slowly under constant stirring at room temperature in Heidolph mixer (650rpm). The composition was stirred for additional 5 min. This phase was slowly added to Carbopol dispersion in insulin aqueous solution under constant mixing at 400rpm. To the obtained system 0.05g triethanolamine (TEA) were added slowly under constant mixing at 400rpm.

### Example 6

### Insulin-containing composition

0.01g Carbopol 974P was dispersed in 1.18 mL of DDW. In a separate container 0.5 g of phospholipids (Phospholipon 90) and 0.02g ceramide were dissolved in 1.48g ethanol and to this solution 1g propylene glycol were added. To the obtained system 5.8mL of insulin aqueous solution (100IU/mL) were added slowly under constant stirring at room temperature in Heidolph mixer (650rpm). The composition was stirred for additional 5 min. This phase was slowly added to Carbopol dispersion in DDW under constant mixing at 400rpm. To the obtained system 0.01g triethanelamine (TEA) were added slowly under constant mixing at 400rpm.

### Example 7

### Dihydroartemisinin-containing compositions

| | |
|---|---|
| Dihydroartemisinin | 23-350mg |
| Phospholipid | 70-250mg |
| Ethanol | 750-1050mg |
| Propylene glycol | 350-1000mg |
| Water to | 3.5g |

Preparation: Phospholipid was dissolved in ethanol and to this solution propylene glycol was added. To the obtained solution DHA was added and the mixture was left at room temperature for 3-4 days. Then DDW was added to the composition slowly under constant stirring. The composition was stirred for additional 15 min.

### Reference Example 8

### Diazepam-containing composition

1 g soy phospholipid was dissolved in a mixture of 3 g ethanol and 9.8 g propylene glycol and to this solution 400mg of diazepam and 2.4 g Labrasol was added. Water (3.4 g) preheated to 40C was added slowly with constant stirring in Heidolph mixer (650rpm). The composition is stirred for additional 15min. The final composition contains 2%w/w diazepam.

### Example 9

### Granisetron HCl-containing composition

50 mg of soy phospholipids were dissolved in 150 mg ethanol. To this solution, 200 mg of propylene glycol and 10mg Labrasol were added and mixed. To the obtained mixture 15 mg of granisetron were added and dissolved. 575 microlitter of DDW (at room temperature) were added very slowly under constant vortexing. The composition is stirred for additional 5 min.

### Example 10

### Granisetron HCl-containing composition

70mg of Phospholipon 90 were dissolved in 150 mg ethanol. To this solution, 230mg propylene glycol were added and mixed. To the obtained mixture, 20mg of granisetron HCl were added and dissolved. 530 microlitter of DDW (preheated to 40C) were added very slowly under constant vortexing. The composition is stirred for additional 15 min.

### Example 11

### Hypoglycemic effect (reduced blood glucose levels) by intranasal administration of insulin

Tables IA and IB detail various compositions of human insulin, which were prepared according to the procedures described in Examples 1-6 above.

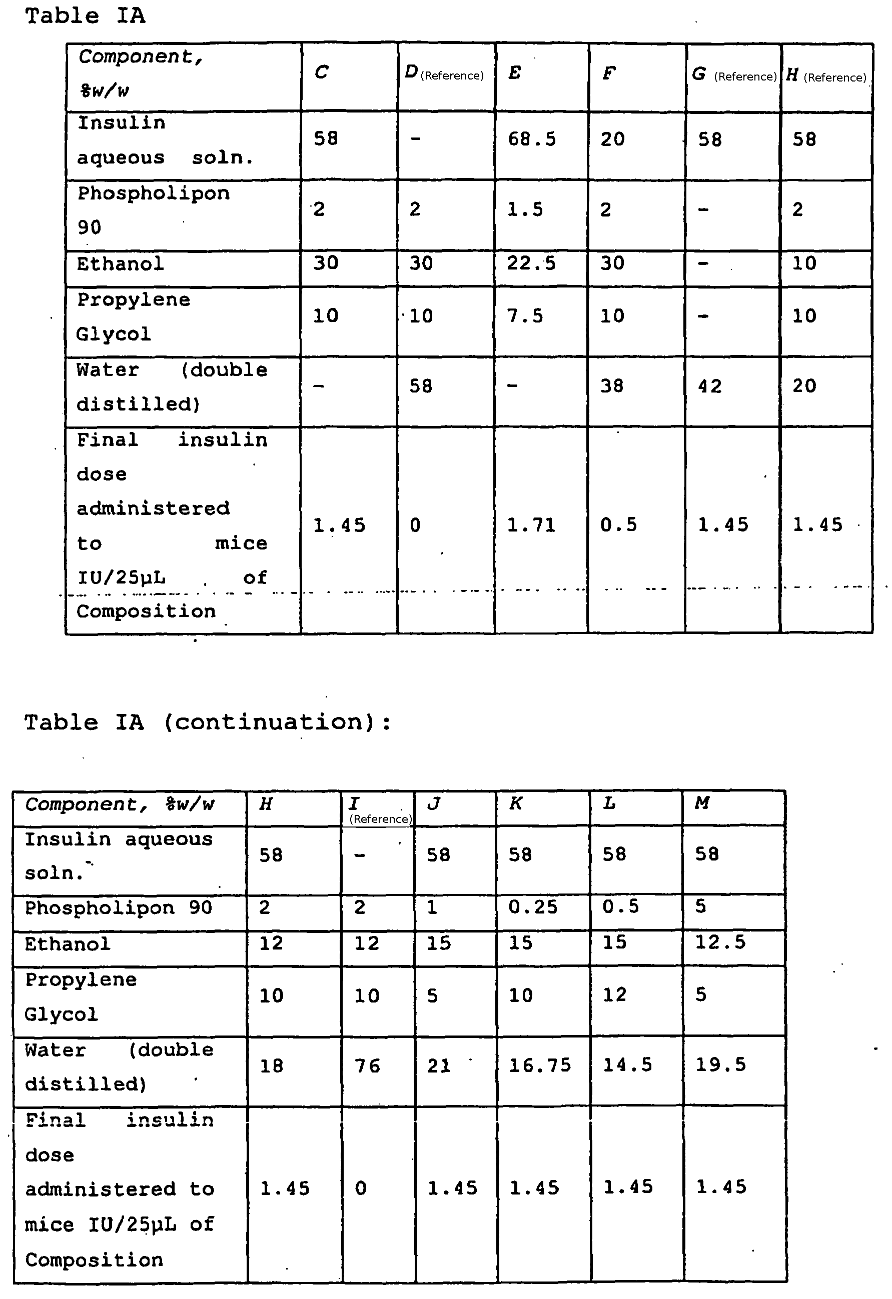

**Table IB**

| *Component*, *% w*/*w* | *N* | *O* | *P* | *Q* | *R* | *S* | *T* | *U* | *V* |
|---|---|---|---|---|---|---|---|---|---|
| Insulin aqueous soln. | 58 | 58 | 58 | 58 | 58 | 58 | 58 | 58 | 58 |
| Phospholipon 90 | 5 | 2 | 9 | 10 | 8 | 1 | 5 | 5 | 1 |
| Cholesterol | - | - | 1 | - | - | 0. 1 | 1.5 | - | - |
| Ceramide | - | - | - | 1 | - | - | - | 0.2 | - |
| Tween 20 | - | - | - | 1.8 | - | - | 6.5 | - | - |
| Ethanol | 15 | 15 | 20 | 20 | 20 | 20 | 18.5 | 14.8 | 12 |
| Propylene Glycol | 10 | 10 | 12 | 9 | 10 | 10 | 10 | 10 | 15 |
| Water (double distilled) | 12 | 15 | - | - | 3.9 | 9. 8 | - | 11.9 | 13.5 |
| Hydroxy-propyl cellulose | - | - | - | 0.2 | 0.1 | | - | - | 0.5 |
| Carbopol | - | - | - | - | - | 0. 1 | 0.5 | 0.1 | - |

The effect of nasal administration of insulin to mice by means of the compositions described in Tables IA and IB was tested as follows.

Experiments were carried out on C75/bl male mice (weight 22-28g). 25 µL of the Compositions (see Figures and Table) were applied to the nasal cavity of the animal under short isofluran anesthesia. The mice have not received food during the experiment. Blood glucose levels were measured by glucose oxidase method using Glucometer Elite (disposable strips). The measurements were performed starting from one hour prior to intranasal administration of Compositions up to a maximum of 8 hours from the administration. Compositions D and I were used as Placebo controls for the Compositions C and H, respectively. Composition G served as the insulin aqueous solution control.

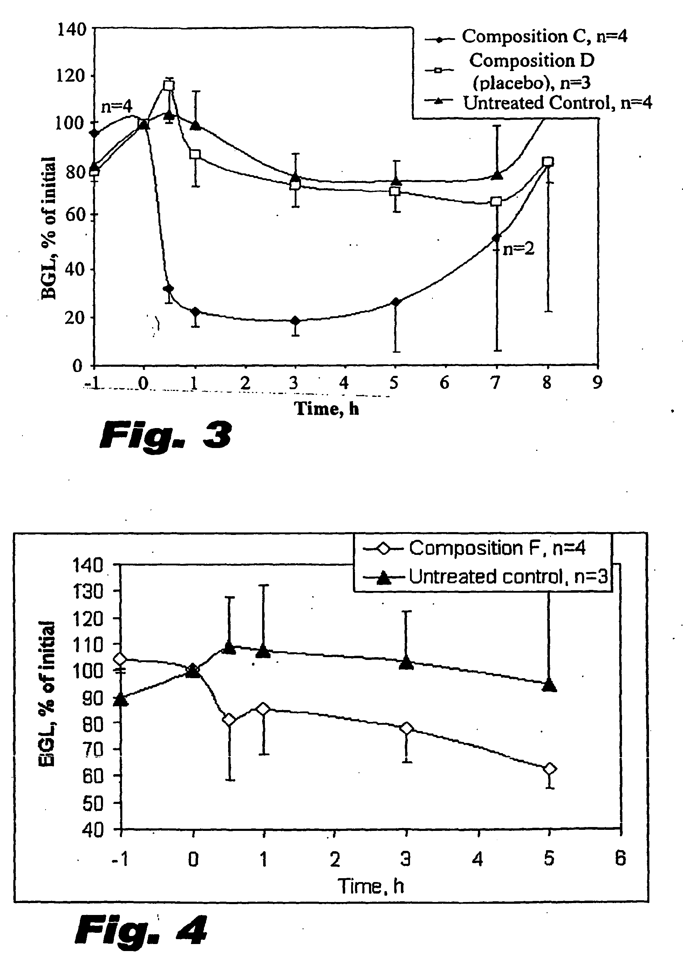

Figures 2-5 present the Blood Glucose Levels (BGL) profiles following administration of various insulin compositions. Administration of compositions D and I (placebo controls), or composition G (aqueous control) had no effect on BGL (Figures 2 and 3). Compositions C, F, N and O significantly improved intranasal insulin absorption reducing the BGL.

### Example 12

### Treatment and prophylaxis of malaria by intranasal administration of dihydroartemisinin (DHA)

Table II details compositions of dihydroartemisinin, which were prepared according to the procedure described in Example 7 above.

**Table II:**

| *Component*, *% w*/*w* | *A* | *B* | *C* | *D* | *E* |
|---|---|---|---|---|---|
| Dihydroartemisinin (DHA) | 0.66 | 0.66 | 0.33 | 0.40 | 10 |
| Phospholipon 90 | 2 | 2 | 5 | 2 | 5 |
| Ethanol | 27 | 20 | 17 | 22 | 28 |
| Propylene Glycol | 10 | 20 | 20 | 15 | 25 |
| Tween 20 | - | 10 | - | 5 | 2 |
| Water (double distilled) | 54.34 | 47.34 | 57.67 | 55.6 | 30 |

The compositions described in Table II were tested as follows.

Experiments were carried out in vivo in ICR female mice infected with 10⁶ erythrocytes parasitized Plasmodium berghei anka, a model of cerebral malaria with striking similarities to the human disease. Infections were monitored using giemsa-stained thin blood smears prepared from tail blood. The animals were treated under isoflurane anesthesia with 10mg DHA/kg/day in a two divided daily doses by two dosage regimens: prophylaxis regimen- starting at 2 days before the infection for a total of 6 days; treatment regimen- starting on day 2 after infection (parasitemia first detected) for a total of 4 days. Mice were either treated by the intranasal administration or by the i.p. injection containing the same DHA doses. Controls included placebo (delivery carrier only) and untreated infected animals. Experiments were conducted in accordance with institutional guidelines for animal care.

Results show that parasites were not detected in the prophylaxis regimen animal group treated with intranasal administration of DHA in the enhancing permeation carrier, but appeared in 74% of mice treated in the same regimen by i.p. DHA injection. In the treatment regimen, 75% of mice which received intranasal DHA survived, in comparison with only 19% in the i.p. treatment group. Isoflurane anesthesia and the administration of the placebo carrier did not affect the development of the disease. All mice in the control groups succumbed to the parasitemia.

In conclusion, it has been shown that DHA intranasal administration from an enhancing permeation carrier, was effective for prophylaxis and treatment of anemic and cerebral malaria in mice.

### Example 13

### Intranasal administration of diazepam

The efficacy of the intranasal administration of the diazepam-containing composition prepared according to Reference Example 8 was tested by means of the following experiments.

Experiments 1: The experiments were carried out on Female Balb/c mice (21-26g). Two experimental groups were used: control (untreated) (n=6) and treated group (n=6). The animals in active treatment group were administered with the Diazepam intranasal Phospholipid ethanolic vesicular compositions 2.9µl in each nose (5mg/kg animal). Half an hour after nasal application, each animal in treated and control groups was IP administered with acetic acid 0.6% (10 ml/kg) and individually housed in cage with a smooth flat floor. Antinociception effect was recorded by counting the number of writhes 5 minutes after injection of acetic acid for period of 10 minutes. A writhe is indicated by abdominal constriction and stretching of at least one hind limb.

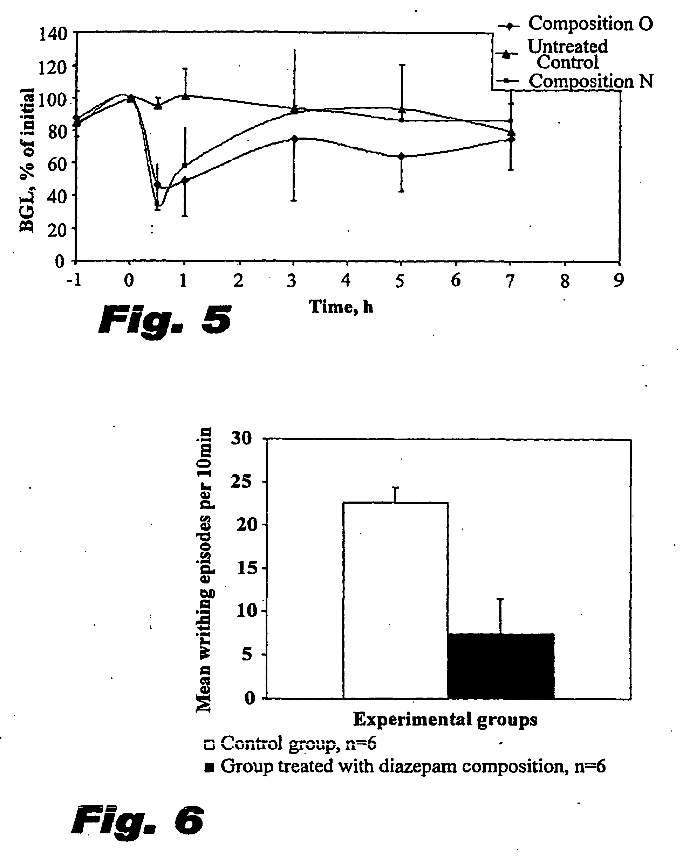

Figure 6 is a bar diagram illustrating the results obtained, which show that intranasal administration of diazepam from the vesicular composition, 0.5 h before acetic acid injection efficiently prevented writhing episodes.

Experiment 2: The experiment was carried out on Female Balb/c mice (21-26g). Two experimental groups were used: control (untreated) (n=6) and treated group (n=6). The animals in active treatment group were administered with the Diazepam intranasal vesicular composition 2.9µl in each nose (5mg/kg animal). Immediately after nasal application (t=0), each animal in treated and control groups was IP administered with acetic acid 0.6% (10 ml/kg) and individually housed in cage with a smooth flat floor. Antinociception was recorded by counting the number of writhes 5 minutes after injection of acetic acid for period of 10 minutes.

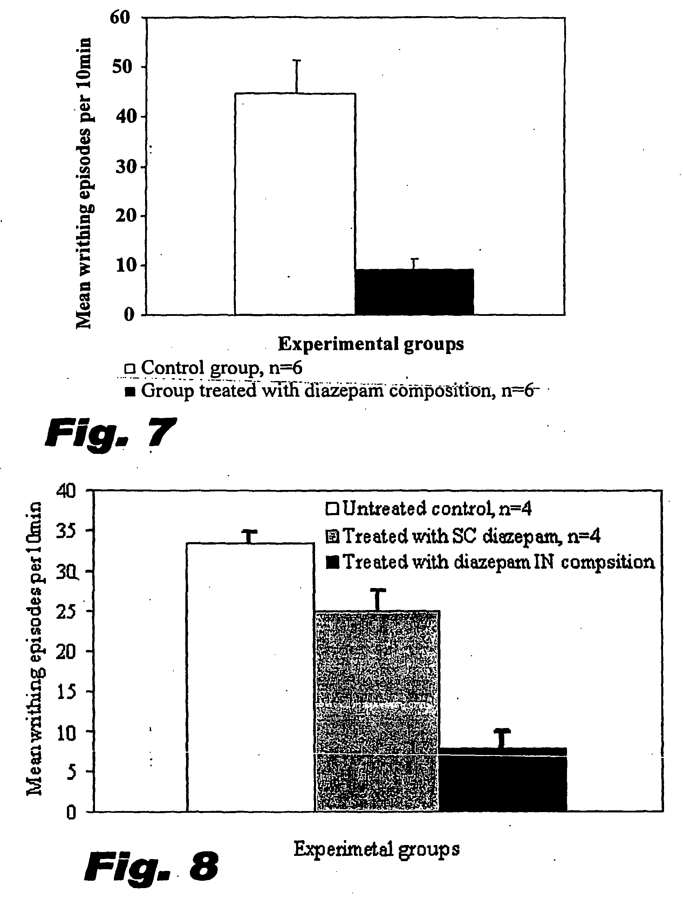

Figure 7 is a bar diagram illustrating the results obtained, which show that intranasal administration of diazepam from the vesicular composition simultaneously with injection of acetic acid solution was efficient in treating writhing episodes.

Experiment 3: The experiments were carried out on Female Balb/c mice (21-26g). Three experimental groups were used: control (untreated) (n=4), mice intranasally administered with the Diazepam IN vesicular composition (2.8µl in each nostril = diazepam dose of 5mg/kg animal) (n=4), and mice subcutaneously administered with the Diazepam solution 0.125 % at dose of 5mg/kg animal (n=4). The animals in active treatment groups were administered with the Diazepam intranasal composition and subcutaneous diazepam. Simultaneously, each animal in treated and control groups was IP administered with acetic acid 0.6% (10 ml/kg) and individually housed in cage with a smooth flat floor. Antinociception was recorded by counting the number of writhes 5 minutes after injection of acetic acid for period of 10 minutes.

Figure 8 is a bar diagram illustrating the results obtained, which show that intranasal administration of diazepam from the vesicular composition, was significantly more efficient in treating writhing episodes as compared to the same dose of the drug administered subcutaneously.

### Example 14

### Intranasal administration of granisetron HCl

Table III details compositions of granisetron, which were prepared according to the procedures described in Examples 9-10 above.

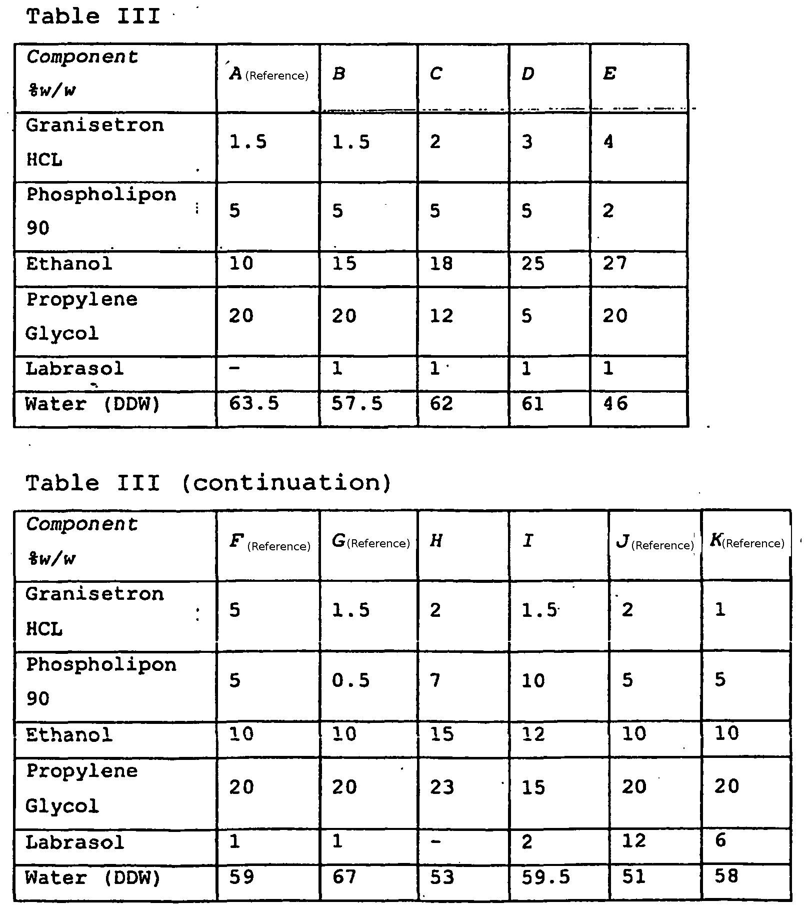

The compositions detailed in Table III were used for the intranasal administration of granisetron hydrochloride to rats and the pharmacodynamic response thereof was evaluated as follows.

Experiments were carried out on Male SD/H rats weighing 200-240 g. The animals were housed individually in cages (23×23×20 cm) in a room with a 12-h light/12-h dark cycle (lights on between 06:00 and 18:00 h) at a constant temperature (27±1 °C) and humidity (50±5%). Pelleted food and water was available ad libitum. Each cage had a wire-mesh floor to permit collection of spilt kaolin and food. Kaolin pellets were prepared according to the methods described Takeda et al. (1993). Briefly, gum Arabic and hydrated aluminum silicate (kaolin- China clay) were mixed together (1:100 on a weight: weight basis) with distilled water to form a thick paste. Pellets of the resulting kaolin mixture were shaped to resemble the dimensions of the rats' normal laboratory diet. The pellets were dried completely at room temperature.

The kaolin pellets were introduced into the cages 3 days prior to drug administration. They were held in identical stainless-steel containers (7×8×3 cm, attached to the side of the cage) to the food pellets. The kaolin and food containers were removed each day (at 10:00 h) and the spilt kaolin and food collected, to determine the rats' consumption, during each 24-h period, up to a total 72 h observation time. Rat weight was also recorded on a daily basis.

Ipecac syrup 5ml/kg was administrated orally and animals returned to the experiment cages. Rats were administrated with intranasal Granisetron HCl Composition B (at a dose of 1.5mg granisetron HCl/kg rat). One hour after intranasal administration of granisetron, Ipecac syrup was given orally using a gavage to treated (n=5) and untreated (control, n=5) animals. Immediately after Ipecac syrup, the animals in the treatment group were administered with an additional dose of intranasal Granisetron hydrochloride followed by drug intranasal administration at regular 12-h intervals for additional 2.5 days. Kaolin and food intake as well as rat weights were measured at 24, 48 and 72 h post- Ipecac.

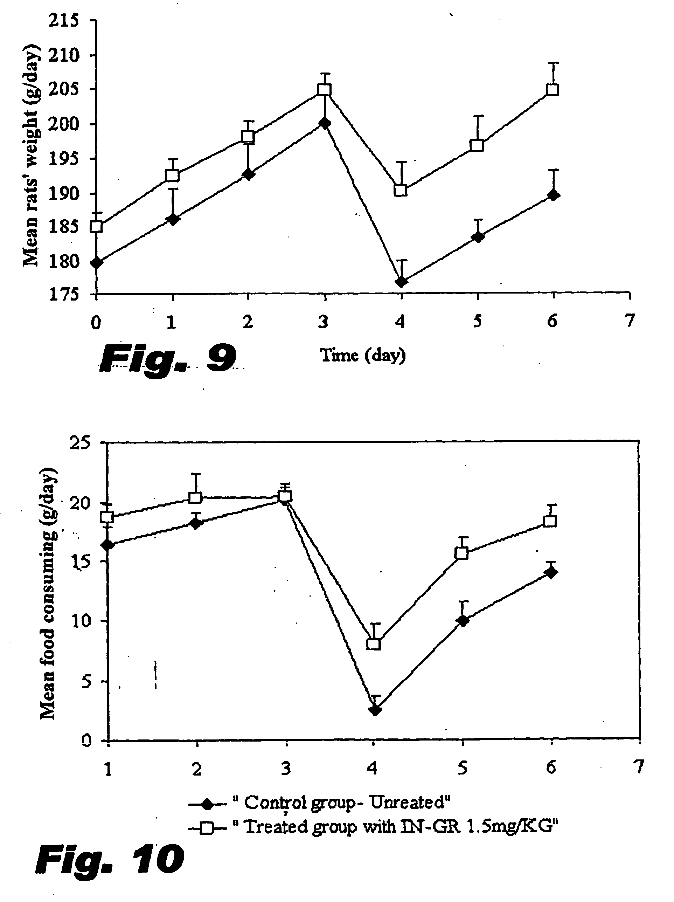

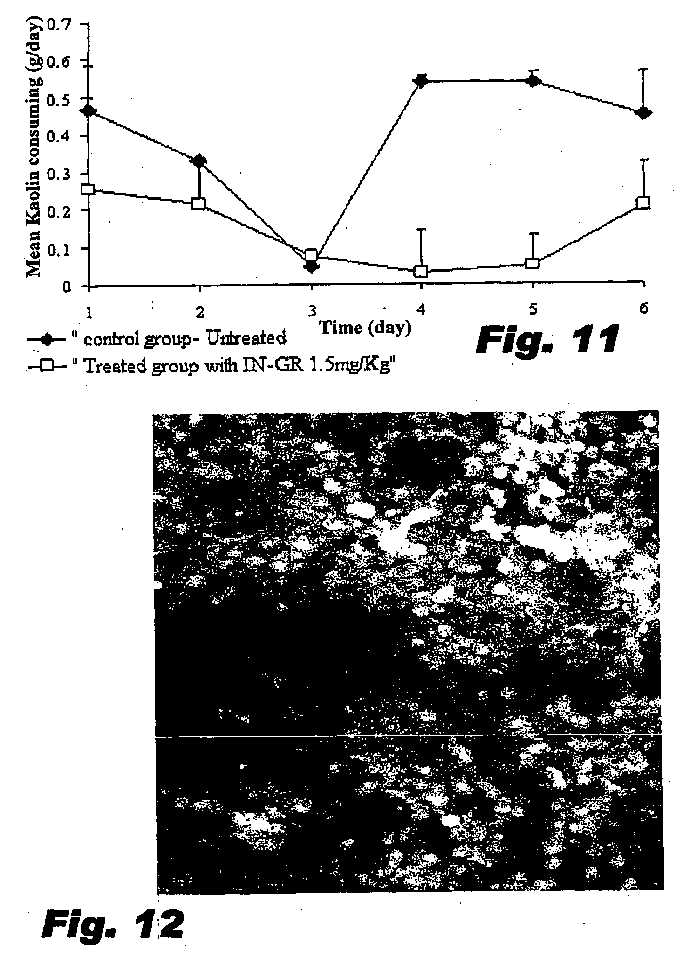

The results collected are represented in Figures 9 to 11. The Results show that intranasal administration of granisetron HCl from composition B, was efficient in preventing weight loss (Fig. 9), stimulating food consumption (Fig. 10) and preventing kaolin consumption (Fig. 11) in rats with Pica syndrome (equivalent to emesis and vomiting in humans).

### Example 15

### Transport of fluorescent probe across nasal mucosa following in vivo administration

Visualization of Rhodamine B (hydrophilic probe, MW 479) permeation across the nasal mucosa using the composition of the invention (containing 0.05% (0.5mg/mL) Rhodamine B) was carried out as follows.

A stock solution of Rhodamine B (2mg/mL) was prepared in water. 50mg of phospholipid were dissolved in 200 mg ethanol. To this solution 100 mg propylene glycol and 10 mg Labrasol were added and mixed. To the obtained mixture 250 microliter of the aforementioned aqueous Rhodamine B solution (2mg/ml) were added slowly with constant stirring. The residual 390 microlitter of DDW were added slowly to the obtained system with constant vortexing. The composition is stirred for additional 5 min. The composition is described in Table IV.

**Table IV**

| *Component* | *Rhodamine B composition %w*/*w* |
|---|---|
| Rhodamine B stock aqueous soln. | 25 |
| Phospholipon 90 | 5 |
| Ethanol | 20 |
| Propylene Glycol | 10 |
| Labrasol | 1 |
| Water (DDW) | 39 |

The composition was applied intranasally to the right nostril of SD/H male 220-250g rats (application volume 100µL) anesthetized i.p. with Ketamine-Xylazine mixture The animals were sacrificed 1/2 hour from the application and the nasal septum with the adjunct epithelial membrane from each animal were carefully removed from the bone. The harvested septum was fixed with 3.8% Formalin in PBS (pH 7.4) for 1 hour in room temperature. The untreated epithelia on the left side of the septum were separated from the septum. The septum with right side epithelia was placed on the slide, covered with cover glass, fixed with tape and observed under CLS microscope (10-40X/0.6 plan Neofluor lens, Zeiss LSM 410 confocal system with an Axiovert 135 inverted microscope).

Figure 12 is a photograph showing that the composition of the invention efficiently delivered rhodamine B across the nasal mucosa (White means the highest fluorescent intensity).

### Example 16

### Granisetron HCL-containing composition in the form of a viscous liquid

700mg of Phospholipon 90 were dissolved in 1500 mg ethanol. To this solution 2300mg of propylene glycol were added and mixed. To the obtained mixture 200mg of granisetron were added and dissolved. 5280 microlitter of DDW (preheated to 40C) were added very slowly under constant mixing in Heidolph mixer (650rpm). The composition was mixed for additional 15 min. To the obtained system 20mg of hydroxypropylcellulose were added slowly and mixed for additional 15 min in Heidolph mixer (650rpm). The resulting composition was left for 30min in room temperature and than mixed for additional 5min.

### Example 17

### Insulin-containing composition in the form of a semi-solid

0.2 g of phospholipon 90 were dissolved in 3g ethanol and to this solution 0.94g propylene glycol were added. The obtained solution was added slowly to 5.8 mL of the aqueous insulin solution (100IU/mL) under constant stirring at room temperature in Heidolph mixer (650rpm). The composition was stirred for additional 5 min. To the obtained system 60 mg of hydroxypropylcellulose were added slowly and mixed for additional 15 min in Heidolph mixer (650rpm). The resulting composition was left for 30min in room temperature and than mixed for additional 10 min. The final semi-solid composition contains 58IU insulin/ g.

### Example 18

### Insulin-containing composition in the form of a gel

0.2g of Carbopol 980 was dispersed in 2.48g DDW in Heidolph mixer (400rpm) followed by a slow addition of 0.2 g of TEA. The mixture was left for 10min in room temperature to obtain the gel phase.

In another container 0.2g of Phospholipin 90 were dissolved in 2g EtOH to this solution 1g of propylene glycol and 0.02g of Vitamin E were added and mixed to obtain clear system in Heidolph mixer (700rpm). The obtained system was stirred for additional 5 min and added slowly to the gel phase under constant mixing at 400rpm. To the obtained semi-solid preparation 3.9mL of insulin aqueous solution containing 250 IU/mL (prepared from dissolving 40.6mg of human insulin powder containing 24IU/mg (Sigma) in DDW) was added. The obtained composition was mixed for additional 5 min. It is notable that insulin solution could be added in each stage of the preparation. The final semi-solid composition contains 97.5IU insulin/ g.

### Example 19 (comparative)

Insulin-containing compositions were prepared, as described in Table V below:

**Table V**

| | *Compositions*, , *%w*/*w* | | |
|---|---|---|---|
| *Component* | *I* | *II* | *III* |
| Insulin aqueous solution 100IU/ml | 63 | 63 | 63 |
| Phospholipon 90 | 2 | 2 | 2 |
| Ethanol | 25 | 10 | 2 |
| Propylene Glycol | 10 | - | - |
| DDW | - | 25 | 33 |
| Final insulin dose administered to mice IU/25µL of Composition | 1.575 IU | 1.575 IU | 1.575 IU |

### Experimental protocol:

Nasal absorption experiments with insulin compositions I, II (control composition containing 10% EtOH) and III (control liposomal composition containing 2% EtOH) were performed in ICR/male mice (7-10Weeks) obtained from (Harlan/Israel). The animals were fasted 1 h prior to an insulin administration and during the experiment time, with free access to water. Compositions were intranasally administered to the animals (12.5µl in each nostril, a total of 25 pi per animal- each nose side), using a pipette with a disposable plastic tip. The nasal insulin formulations were administered at time=0h following a short isofluran anesthesia. The total amount of insulin delivered nasally to each animal, was 1.575 IU. Blood glucose levels were measured by glucose oxidase method using Glucometer Elite (disposable strips). The measurements were performed starting from one hour prior to intranasal administration of Compositions up to 6 hours from the administration.

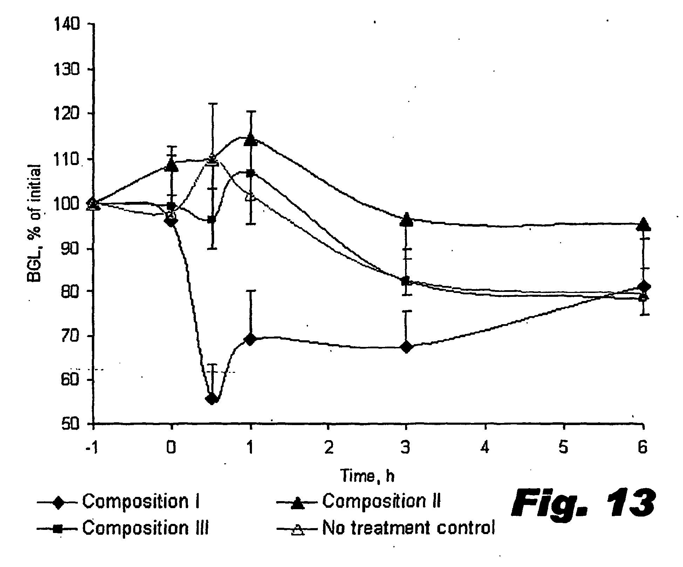

The results presented in Figure 13 show that Composition I efficiently reduced blood glucose levels, while administration of Compositions II and III (controls) had no effect on BGL.

### Example 20

### Buspirone HCl-containing composition

The following compositions were prepared:

| *Component*, *%w*/*w* | | |
|---|---|---|
| | *A* | *B* |
| Buspirone HCL | 1 | 2 |
| Phospholipon 90 | 2 | 2 |
| Ethanol | 20 | 25 |
| Propylene Glycol | 10 | - |
| Vitamin E | 0.2 | 0.2 |
| Carbopol 980 | 1 | - |
| Triethanolamine (TEA) | 1 | - |
| Water (DDW) | 64.8 | 70.8 |

### Preparation method for Buspirone Composition A:

0.1g of Carbopol 980 was dispersed in 2.48g DDW in Heidolph mixer (400rpm) to this dispersion 1g of EtOH was added under constant mixing followed by a slow addition of 0.1 g of TEA. The mixture was left for 10min in room temperature to obtain the gel phase.

In another container 0.2g of Phospholipin 90 were dissolved in 1g EtOH to this solution 1g of propylene glycol and 0.02g of Vitamin E were added and mixed to obtain clear system. To this system 0.1g of buspirone HCl dissolved in 4g DDW were slowly added under constant stirring at room temperature in Heidolph mixer (700rpm). The obtained system was stirred for additional 5 min and added slowly to the gel phase under constant mixing at 400rpm. The obtained composition A was mixed for additional 5 min.

### Preparation method for Buspirone Composition A:

0.2g of Phospholipin 90 were dissolved in 2.5g EtOH; to this solution 0.02g of Vitamin E were added and mixed to obtain clear system. To this system, 0.2g of buspirone HCl dissolved in 7.08g DDW were slowly added under constant stirring at room temperature in Heidolph mixer (700rpm). The obtained system was stirred for additional 5 min.

### Example 21

### Insulin-containing composition

0.2g mg of phospholipids (Phospholipon 90) were dissolved in 1.5g ethanol and to this solution 0.5g propylene glycol were added.
Insulin aqueous solution containing 250 IU/mL insulin was prepared by dissolving 81.25mg of human insulin powder containing 24IU/mg (Sigma) in 7.8 mL DDW. The obtained insulin aqueous solution was added slowly under constant stirring at room temperature to the previously prepared phospholipid solution. The composition is stirred for additional 5 min. The final composition contains 195 IU insulin/g.

### Example 22

### Glatiramer acetate -containing composition

The following compositions were prepared:

| *Component*, *%w*/*w* | | | |
|---|---|---|---|
| | *A* | *B* | *C* |
| Glatiramer acetate | 1 | 2 | 2 |
| Soy phospholipids | 2 | 2 | 3 |
| Ethanol | 20 | 25 | 15 |
| Propylene Glycol | 10 | - | 10 |
| Vitamin E | 0.2 | 0.2 | 0.2 |
| Carbopol 980 | 1 | - | 0.1 |
| Triethanolamine (TEA) | 1 | - | 0.1 |
| Water (DDW) | 64.8 | 70.8 | 69.6 |

### Example 23

### Paroxetine -containing composition

The following compositions were prepared:

| *Component*, *%w*/*w* | | |
|---|---|---|
| | *A* | *B* |
| Paroxetine | 0.5 | 1 |
| Phosphatydylcholine | 2.5 | 3 |
| Ethanol | 23 | 15 |
| Propylene Glycol | 10 | 15 |
| Vitamin E | 0.2 | 0.2 |
| Labrasol | 1 | - |
| Water (DDW) | 62.8 | 65.8 |

### Example 24

### Rivastigmine -containing composition

The following compositions were prepared:

| *Component*, *%w*/*w* | | |
|---|---|---|
| | *A* | *B* |
| Rivastigmine tartrate | 0.5 | 0.75 |
| Soy Phospholipid | 2 | 5 |
| Ethanol | 12 | 20 |
| Propylene Glycol | 10 | 15 |
| Water (DDW) | 75.5 | 59.25 |

### Example 25

### Sibutramine -containing composition

The following compositions were prepared:

| *Component*, *%w*/*w* | | |
|---|---|---|
| | *A* | *B* |
| Sibutramine | 1 | 1.5 |
| Phospholipon 90 | 5 | 2 |
| Ethanol | 14 | 22 |
| Propylene Glycol | 15 | - |
| Vitamin E | 0.2 | - |
| Labrasol | 1 | - |
| Water (DDW) | 63.8 | 74.5 |

## Claims

1. Use of phospholipids, one or more C2-C4 alcohols and water in the preparation of a vesicular composition adapted for intranasal administration of an active agent, wherein the concentrations of said phospholipids and said one or more alcohols in said composition are in the ranges of 0.2 to 10% and 12 to 30% by weight, respectively, with the water content of said composition being not less than 30% by weight.

2. Use according to claim 1, wherein the composition furthermore contains one or more water- miscible polyols, and wherein the concentration of said one or more polyols in said composition is in the range of 1 to 30% by weight.

3. Use according to claim 2, wherein the C2-C4 alcohol is ethanol and the polyol is propylene glycol.

4. Use according to any one of claims 1 to 3, wherein the weight ratio between the C2-C4 alcohol and the phospholipids is not less than 2:1.

5. Use according to any one of claims 1 to 4, wherein said composition is a composition for treating and/or preventing emesis, diabetes, malaria, depression. Alzheimer's disease, multiple sclerosis, hot flushes symptoms and obesity.

6. Use according to claim 1, wherein the composition comprises a therapeutically effective amount of an anti-emetic agent.

7. Use according to claim 6, wherein the anti-emetic agent is granisetron or a pharmaceutically acceptable salt thereof.

8. Use according to claim 1, wherein the composition comprises a therapeutically effective amount of an anti-diabetic agent.

9. Use according to claim 8, wherein the anti-diabetic agent is insulin.

10. Use according to claim 1, wherein the composition comprises a therapeutically effective amount of an anti-malaria agent.

11. Use according to claim 10, wherein the anti-malaria agent is dihydroartemisinin.

12. Use according to claim 1, wherein the composition comprises a therapeutically effective amount of an anti-anxiety and/or anticonvulsant agent.

13. Use according to claim 12, wherein the anti-anxiety and/or anticonvulsant agent is selected from the group consisting of diazepam and buspirone hydrochloride.

14. Use according to claim 1, wherein the composition comprises a therapeutically effective amount of an anti-obesity agent.

15. Use according to claim 14, wherein the anti-obesity agent is sibutramine or a pharmaceutically acceptable salt thereof.

16. Use according to claim 1, wherein the composition comprises a therapeutically effective amount of an antidepressant or anti-hot flashes agent.

17. Use according to claim 16, wherein the antidepressant or anti-hot flashes agent is paroxetine or a pharmaceutically acceptable salt thereof.

18. Use according to claim 1, wherein the composition comprises a therapeutically effective amount of an anti-multiple sclerosis agent.

19. Use according to claim 18, wherein the anti-multiple sclerosis agent is glatiramer acetate.

20. Use according to claim 1, wherein the composition comprises a therapeutically effective amount of an anti-dementia agent.

21. Use according to claim 20, wherein the anti-dementia agent is rivastigmine or a pharmaceutically acceptable salt thereof.

22. Use according to claim 1, wherein the composition comprises a therapeutically effective amount of an analgesic agent.

23. Use according to claim 22, wherein said analgesic agent is tramadol.

24. A pharmaceutical composition for intranasal administration of an active agent, comprising said active agent, water, phospholipids and one or more C2-C4 alcohols, wherein the concentrations of said phospholipids and said one or more alcohols are in the ranges of 0.2 to 10% and 12 to 30% by weight, respectively, with the water content of said composition being not less than 30% by weight, said composition being a vesicular composition, wherein the active agent is selected from the group consisting of granisetron and pharmaceutically acceptable salts thereof, insulin, diazepam, sibutramine and pharmaceutically acceptable salts thereof, paroxetine and pharmaceutically acceptable salts thereof, glatiramer acetate, rivastigmine and pharmaceutically acceptable salts thereof, tramadol , and buspirone and pharmaceutically acceptable salts thereof.

25. A pharmaceutical composition for intranasal administration according to claim 24, which further comprises one or more water miscible polyols at a concentration of 1 to 30% by weight.

26. A pharmaceutical composition according to claim 24 or 25, wherein the active agent is granisetron or a pharmaceutically acceptable salt thereof.

27. A pharmaceutical composition according to claim 24 or 25, wherein the active agent is insulin.

28. A pharmaceutical composition according to claim 24 or 25, wherein the active agent is diazepam.

29. A pharmaceutical composition according to claim 24 or 25, wherein the active agent is sibutramine or a pharmaceutically acceptable salt thereof.

30. A pharmaceutical composition according to claim 24 or 25, wherein the active agent is paroxetine or a pharmaceutically acceptable salt thereof.

31. A pharmaceutical composition according to claim 24 or 25, wherein the active agent is glatiramer acetate.

32. A pharmaceutical composition according to claim 24 or 25, wherein the active agent is rivastigmine or a pharmaceutically acceptable salt thereof.

33. A pharmaceutical composition according to claim 24 or 25, wherein the active agent is tramadol.

34. A pharmaceutical composition according to claim 24 or 25, wherein the active agent is buspirone or a pharmaceutically acceptable salt thereof.

## Patentansprüche

1. Verwendung von Phospholipiden, eines oder mehrerer C2-C4-Alkohole und Wasser bei der Herstellung einer zur intranasalen Verabreichung eines Wirkstoffes angepassten vesikulären Zusammensetzung, wobei die Konzentrationen der Phospholipide und des einen oder der mehreren Alkohole in der Zusammensetzung in den Bereichen von 0,2 bis 10 Gew.-% bzw. 12 bis 30 Gew.-% liegen, wobei der Wassergehalt der Zusammensetzung nicht weniger als 30 Gew.-% beträgt.

2. Verwendung nach Anspruch 1, wobei die Zusammensetzung weiter ein oder mehrere mit Wasser mischbare Polyole enthält und wobei die Konzentration des einen oder der mehreren Polyole in der Zusammensetzung im Bereich von 1 bis 30 Gew.-% liegt.

3. Verwendung nach Anspruch 2, wobei der C2-C4-Alkohol Ethanol ist und das Polyol Propylenglykol ist.

4. Verwendung nach einem der Ansprüche 1 bis 3, wobei das Gewichtsverhältnis zwischen dem C2-C4-Alkohol und den Phospholipiden nicht weniger als 2:1 beträgt.

5. Verwendung nach einem der Ansprüche 1 bis 4, wobei die Zusammensetzung eine Zusammensetzung zur Behandlung und/oder Prävention von Erbrechen, Diabetes, Malaria, Depression, Alzheimer-Krankheit, Multipler Sklerose, Symptomen fliegender Hitze und Adipositas ist.

6. Verwendung nach Anspruch 1, wobei die Zusammensetzung eine therapeutisch wirksame Menge eines Antiemetikums umfasst.

7. Verwendung nach Anspruch 6, wobei das Antiemetikum Granisetron oder ein pharmazeutisch verträgliches Salz davon ist.

8. Verwendung nach Anspruch 1, wobei die Zusammensetzung eine therapeutisch wirksame Menge eines Antidiabetikums umfasst.

9. Verwendung nach Anspruch 8, wobei das Antidiabetikum Insulin ist.

10. Verwendung nach Anspruch 1, wobei die Zusammensetzung eine therapeutisch wirksame Menge eines Antimalariamittels umfasst.

11. Verwendung nach Anspruch 10, wobei das Antimalariamittel Dihydroartemisinin ist.

12. Verwendung nach Anspruch 1, wobei die Zusammensetzung eine therapeutisch wirksame Menge eines angstlösenden und/oder krampflösenden Mittels umfasst.

13. Verwendung nach Anspruch 12, wobei das angstlösende und/oder krampflösende Mittel aus der Gruppe bestehend aus Diazepam und Buspironhydrochlorid ausgewählt ist.

14. Verwendung nach Anspruch 1, wobei die Zusammensetzung eine therapeutisch wirksame Menge eines Mittels gegen Adipositas umfasst.

15. Verwendung nach Anspruch 14, wobei das Mittel gegen Adipositas Sibutramin oder ein pharmazeutisch verträgliches Salz davon ist.

16. Verwendung nach Anspruch 1, wobei die Zusammensetzung eine therapeutisch wirksame Menge eines Antidepressivums oder Mittels gegen fliegende Hitze umfasst.

17. Verwendung nach Anspruch 16, wobei das Antidepressivum oder Mittel gegen fliegende Hitze Paroxetin oder ein pharmazeutisch verträgliches Salz davon ist.

18. Verwendung nach Anspruch 1, wobei die Zusammensetzung eine therapeutisch wirksame Menge eines Mittels gegen Multiple Sklerose umfasst.

19. Verwendung nach Anspruch 18, wobei das Mittel gegen Multiple Sklerose Glatirameracetat ist.

20. Verwendung nach Anspruch 1, wobei die Zusammensetzung eine therapeutisch wirksame Menge eines Antidemenzmittels umfasst.

21. Verwendung nach Anspruch 20, wobei das Antidemenzmittel Rivastigmin oder ein pharmazeutisch verträgliches Salz davon ist.

22. Verwendung nach Anspruch 1, wobei die Zusammensetzung eine therapeutisch wirksame Menge eines Analgetikums umfasst.

23. Verwendung nach Anspruch 22, wobei das Analgetikum Tramadol ist.

24. Ein Arzneimittel zur intranasalen Verabreichung eines Wirkstoffes, umfassend den Wirkstoff, Wasser, Phospholipide und einen oder mehrere C2-C4-Alkohole, wobei die Konzentrationen der Phospholipide und des einen oder der mehreren Alkohole in den Bereichen von 0,2 bis 10 Gew.-% bzw. 12 bis 30 Gew.-% liegen, wobei der Wassergehalt der Zusammensetzung nicht weniger als 30 Gew.-% beträgt, wobei die Zusammensetzung eine vesikuläre Zusammensetzung ist, wobei der Wirkstoff aus der Gruppe bestehend aus Granisetron und pharmazeutisch verträglichen Salzen davon, Insulin, Diazepam, Sibutramin und pharmazeutisch verträglichen Salzen davon, Paroxetin und pharmazeutisch verträglichen Salzen davon, Glatirameracetat, Rivastigmin und pharmazeutisch verträglichen Salzen davon, Tramadol, und Buspiron und pharmazeutisch verträglichen Salzen davon ausgewählt ist.

25. Ein Arzneimittel zur intranasalen Verabreichung nach Anspruch 24, welches weiter ein oder mehrere mit Wasser mischbare Polyole in einer Konzentration von 1 bis 30 Gew.-% umfasst.

26. Ein Arzneimittel nach Anspruch 24 oder 25, wobei der Wirkstoff Granisetron oder ein pharmazeutisch verträgliches Salz davon ist.

27. Ein Arzneimittel nach Anspruch 24 oder 25, wobei der Wirkstoff Insulin ist.

28. Ein Arzneimittel nach Anspruch 24 oder 25, wobei der Wirkstoff Diazepam ist.

29. Ein Arzneimittel nach Anspruch 24 oder 25, wobei der Wirkstoff Sibutramin oder ein pharmazeutisch verträgliches Salz davon ist.

30. Ein Arzneimittel nach Anspruch 24 oder 25, wobei der Wirkstoff Paroxetin oder ein pharmazeutisch verträgliches Salz davon ist.

31. Ein Arzneimittel nach Anspruch 24 oder 25, wobei der Wirkstoff Glatirameracetat ist.

32. Ein Arzneimittel nach Anspruch 24 oder 25, wobei der Wirkstoff Rivastigmin oder ein pharmazeutisch verträgliches Salz davon ist.

33. Ein Arzneimittel nach Anspruch 24 oder 25, wobei der Wirkstoff Tramadol ist.

34. Ein Arzneimittel nach Anspruch 24 oder 25, wobei der Wirkstoff Buspiron oder ein pharmazeutisch verträgliches Salz davon ist.

## Revendications

1. Utilisation de phospholipides, d'un ou plusieurs alcools en C₂ à C₄ et d'eau dans la préparation d'une composition vésiculaire adaptée à une administration intranasale d'un agent actif, où les concentrations desdits phospholipides et desdits un ou plusieurs alcools dans ladite composition se situent, respectivement, dans les plages de 0,2 à 10 % et de 12 à 30 % en poids, avec la teneur en eau de ladite composition n'étant pas inférieure à 30 % en poids.

2. Utilisation selon la revendication 1, où la composition contient en outre un ou plusieurs polyols miscibles à l'eau, et où la concentration desdits un ou plusieurs polyols dans ladite composition se situe dans la plage de 1 à 30 % en poids.

3. Utilisation selon la revendication 2, où l'alcool en C₂ à C₄ est l'éthanol et le polyol est le propylène glycol.

4. Utilisation selon l'une quelconque des revendications 1 à 3, où le rapport pondéral entre l'alcool en C₂ à C₄ et les phospholipides n'est pas inférieur à 2/1.

5. Utilisation selon l'une quelconque des revendications 1 à 4, où ladite composition est une composition destinée au traitement et/ou à la prévention des vomissements, du diabète, du paludisme, de la dépression, de la maladie d'Alzheimer, de la sclérose en plaques, des symptômes de bouffées de chaleur et de l'obésité.

6. Utilisation selon la revendication 1, où la composition comprend une quantité thérapeutiquement efficace d'un agent antiémétique.

7. Utilisation selon la revendication 6, où l'agent antiémétique est le granisétron ou un sel pharmaceutiquement acceptable de celui-ci.

8. Utilisation selon la revendication 1, où la composition comprend une quantité thérapeutiquement efficace d'un agent antidiabétique.

9. Utilisation selon la revendication 8, où l'agent antidiabétique est l'insuline.

10. Utilisation selon la revendication 1, où la composition comprend une quantité thérapeutiquement efficace d'un agent antimalaria.

11. Utilisation selon la revendication 10, où l'agent antimalaria est la dihydroartémisinine.

12. Utilisation selon la revendication 1, où la composition comprend une quantité thérapeutiquement efficace d'un agent anxiolytique et/ou anticonvulsivant.

13. Utilisation selon la revendication 12, où l'agent anxiolytique et/ou anticonvulsivant est choisi dans le groupe constitué du diazépam et du chlorhydrate de buspirone.

14. Utilisation selon la revendication 1, où la composition comprend une quantité thérapeutiquement efficace d'un agent anti-obésité.

15. Utilisation selon la revendication 14, où l'agent anti-obésité est la sibutramine ou un sel pharmaceutiquement acceptable de celle-ci.

16. Utilisation selon la revendication 1, où la composition comprend une quantité thérapeutiquement efficace d'un agent antidépresseur ou anti-bouffées de chaleur.

17. Utilisation selon la revendication 16, où l'agent antidépresseur ou anti-bouffées de chaleur est la paroxétine ou un sel pharmaceutiquement acceptable de celle-ci.

18. Utilisation selon la revendication 1, où la composition comprend une quantité thérapeutiquement efficace d'un agent anti-sclérose en plaques.

19. Utilisation selon la revendication 18, où l'agent anti-sclérose en plaques est l'acétate de glatiramère.

20. Utilisation selon la revendication 1, où la composition comprend une quantité thérapeutiquement efficace d'un agent anti-démence.

21. Utilisation selon la revendication 20, où l'agent anti-démence est la rivastigmine ou un sel pharmaceutiquement acceptable de celle-ci.

22. Utilisation selon la revendication 1, où la composition comprend une quantité thérapeutiquement efficace d'un agent analgésique.

23. Utilisation selon la revendication 22, où ledit agent analgésique est le tramadol.

24. Composition pharmaceutique pour une administration intranasale d'un agent actif, comprenant ledit agent actif, de l'eau, des phospholipides et un ou plusieurs alcools en C₂ à C₄, dans laquelle les concentrations desdits phospholipides et desdits un ou plusieurs alcools se situent, respectivement, dans les plages de 0,2 à 10 % et de 12 à 30 % en poids, avec la teneur en eau de ladite composition n'étant pas inférieure à 30 % en poids, ladite composition étant une composition vésiculaire, où l'agent actif est choisi dans le groupe constitué de granisétron et de sels pharmaceutiquement acceptables de celui-ci, d'insuline, de diazépam, de sibutramine et de sels pharmaceutiquement acceptables de celle-ci, de paroxétine et de sels pharmaceutiquement acceptables de celle-ci, d'acétate de glatiramère, de rivastigmine et de sels pharmaceutiquement acceptables de celle-ci, de tramadol et de buspirone et de sels pharmaceutiquement acceptables de celle-ci.

25. Composition pharmaceutique pour une administration intranasale selon la revendication 24, qui comprend en outre un ou plusieurs polyols miscibles à l'eau à une concentration de 1 à 30 % en poids.

26. Composition pharmaceutique selon la revendication 24 ou 25, dans laquelle l'agent actif est le granisétron ou un sel pharmaceutiquement acceptable de celui-ci.

27. Composition pharmaceutique selon la revendication 24 ou 25, dans laquelle l'agent actif est l'insuline.

28. Composition pharmaceutique selon la revendication 24 ou 25, dans laquelle l'agent actif est le diazépam.

29. Composition pharmaceutique selon la revendication 24 ou 25, dans laquelle l'agent actif est la sibutramine ou un sel pharmaceutiquement acceptable de celle-ci.

30. Composition pharmaceutique selon la revendication 24 ou 25, dans laquelle l'agent actif est la paroxétine ou un sel pharmaceutiquement acceptable de celle-ci.

31. Composition pharmaceutique selon la revendication 24 ou 25, dans laquelle l'agent actif est l'acétate de glatiramère.

32. Composition pharmaceutique selon la revendication 24 ou 25, dans laquelle l'agent actif est la rivastigmine ou un sel pharmaceutiquement acceptable de celle-ci.

33. Composition pharmaceutique selon la revendication 24 ou 25, dans laquelle l'agent actif est le tramadol.

34. Composition pharmaceutique selon la revendication 24 ou 25, dans laquelle l'agent actif est la buspirone ou un sel pharmaceutiquement acceptable de celle-ci.
